# EUROPEAN PATENT SPECIFICATION

(11) **EP 3 790 648 B1**
(45) Date of publication and mention of the grant of the patent: **21.08.2024**
(21) Application number: 19723375.2
(22) Date of filing: 08.05.2019
(51) Int. Cl.: B01D 61/00

(54) **METHOD FOR ENRICHING AQUEOUS ETHANOLIC SOLUTION IN ETHANOL**
VERFAHREN ZUR ANREICHERUNG EINER WÄSSRIGEN ETHANOLISCHEN LÖSUNG IN ETHANOL
PROCÉDÉ D'ENRICHISSEMENT D'UNE SOLUTION ÉTHANOLIQUE AQUEUSE EN ÉTHANOL

(30) Priority: 09.05.2018 DK PA201870284
(43) Date of publication of application: 17.03.2021
(73) Proprietor: Aquaporin A/S, 2800 Kongens Lyngby (DK)
(72) Inventor: BRAEKEVELT, Sylvie, Frederiksberg 2000 (DK)
(74) Representative: Nordic Patent Service A/S
(86) International application number: PCT/EP2019/061815
(87) International publication number: WO 2019/215226

(56) References cited:
- WO-A1-2017/137361
- WO-A2-2016/210337
- US-A1- 2010 155 333
- XIWANG ZHANG ET AL: "A novel ethanol dehydration process by forward osmosis", CHEMICAL ENGINEERING JOURNAL, vol. 232, 1 October 2013 (2013-10-01), AMSTERDAM, NL, pages 397 - 404, XP055609017, ISSN: 1385-8947, DOI: 10.1016/j.cej.2013.07.106
- ALAN AMBROSI ET AL: "Transport of Components in the Separation of Ethanol from Aqueous Dilute Solutions by Forward Osmosis", INDUSTRIAL & ENGINEERING CHEMISTRY RESEARCH, vol. 57, no. 8, 28 February 2018 (2018-02-28), pages 2967 - 2975, XP055609019, ISSN: 0888-5885, DOI: 10.1021/acs.iecr.7b04944

## Description

### TECHNICAL FIELD

The disclosure relates to a method for enriching an aqueous ethanolic solution in ethanol.

### BACKGROUND

Dealcoholization is a well-known industrial process in which alcoholic beverages such as beer and wine are reduced in their alcohol concentration. The traditional alcohol separation processes from brews are heat treatment processes, such as evaporation and distillation. As ethanol is more volatile compared to water, it is logical to remove ethanol from the brews by heating. However, the product significantly differs with the regular beer in terms of taste and flavor as some volatile components are thermally degraded during the dealcoholization process.

As the heat treatment processes lack sensorial satisfaction due to high loss of volatile aroma compounds accompanying the removed alcohol, membrane-based separation processes have emerged in this field as a type of ethanol-selective separation process to produce low alcoholic beer or wine with acceptable aroma and taste.

Reverse Osmosis (RO) can be a promising alternative for dealcoholization processes replacing the thermal-based processes because it can remove alcohol under mild temperature. The low molecular weight molecules, such as ethanol and water, permeate across the membrane while the taste and nutritive components of beverages are retained in the product. Since RO is operated under low operating temperature, it offers advantages over traditional distillation which include reduction of energy consumption, high quality of beverage, low damage to temperature sensitive compounds, and alcohol removal without phase change (M. Catarino, A. Mendes, L.M. Madeira, A. Ferreira: Alcohol removal from beer by reverse osmosis, Separation Science and Technology, 42 (13) (2007), pp. 3011-3027).

Membranes of cellulose acetate generally show a good permeation across the membrane of ethanol. Thus, it has been reported that the Alfa Laval membrane DSS-CA995P is capable of dealcoholizing beer having a concentration of 5.26% to an alcohol concentration in the final product (retentate) of about 0.5 % (M. Catarino, A. Mendes, L.M. Madeira, A. Ferreira: Alcohol removal from beer by reverse osmosis, Separation Science and Technology, 42 (13) (2007), pp. 3011-3027)). For a polyester-sulfone membrane it has been shown that a stout beer having an initial alcohol concentration of 6.6% can be reduced to an alcohol concentration of about 0% at a pressure of 4.9 bar and temperature of 20°C (B.M. Alcantara, D.R. Marques, M.M. Chinellato, L.B. Marchi, S.C. da Costa, A.R.G. Monteiro: Assessment of quality and production process of a non-alcoholic stout beer using reverse osmosis, Journal of the Institute of Brewing, 122 (4) (2016), pp. 714-718).

Nanofiltration (NF) is a pressure driven membrane process that uses semipermeable membranes with slightly larger pores than RO membranes, which results in higher fluxes than RO. Experiments on the nanofiltration membrane XN45 from Trisep have shown that water and ethanol permeated through the membrane resulting in a permeate with similar alcoholic content as the original wine. The alcoholic content of permeate was 10.75% ABV (at 20 bar and 30 °C) which was slightly lower than the original wine (12.81% ABV) while the rejection was 9.7%. Furthermore, the valuable components such as sugar, total acid, total extract and sugarless extract were increased to about two times larger from the initial concentrations with low aroma loss (S. Banvolgyi, I. Kiss, E. Bekassy-Molnar, G. Vatai: Concentration of red wine by nanofiltration, Desalination, 198 (1) (2006), pp. 8-15).

Comparison of NF and RO membranes for dealcoholization of wine containing 12% ABV were conducted under 16 bar and 30 °C of feed pressure and operating temperature, respectively (M. Catarino, A. Mendes: Dealcoholizing wine by membrane separation processes, Innovative Food Science and Emerging Technologies, 12 (3) (2011), pp. 330-337). The results showed that NF membranes exhibited higher flux than RO membrane but low ethanol rejection (7-10%). NF membranes showed the effectiveness in dealcoholization of wine due to their high ethanol permeability, high rejection of aroma compounds, and promising organoleptic properties of the product.

In the dealcoholization via dialysis, ethanol is removed using the principle of selective diffusion through a semipermeable membrane. Alcohol diffuses through the membrane from e.g. beer into water as a result of a concentration gradient between both solutions. It has been demonstrated that a 8 µm thick Cuprophane hollow fiber membrane is capable of reducing the ethanol concentration from 3.8% to 0.35% w/w when operated at 2 bar and 5 °C (I. Leskošek, M. Mitrović, V. Nedović: Factors influencing alcohol and extract separation in beer dialysis, World Journal of Microbiology and Biotechnology, 11 (5) (1995), pp. 512-514) .

In the process of dealcoholization of beverages using osmotic distillation (OD), microporous hydrophobic membranes are utilized. An ethanolic feed is contacted with the surface of the membrane at atmospheric pressure and room temperature, while the opposite side of the membrane is contacted to a stripping solution flowed in counter-current mode. As ethanol is permeating through the membrane, the stripping solution is tasked to capture the ethanol. Osmotic distillation may also be referred to as membrane contactor, isothermal membrane distillation, or evaporative pertraction. Among the successful experiments reported in the literature are L. Liguori, P. Russo, D. Albanese, M. Di Matteo: Evolution of quality parameters during red wine dealcoholization by osmotic distillation, Food Chemistry, 140 (1-2) (2013), pp. 68-75. They use a Liqui-cel 1 × 5.5, PP hollow fiber module as the membrane, an Aglianico red wine as the feed, and water as the stripping solution. The wine had an initial alcohol concentration 13% and was during a process time of 255 h reduced to 0.19%. The same membrane type has successfully been used on Italian lager beer (reduction for 5% to 0.89%), craft beer (reduction from 4.3% to 0.7%), weiss beer (reduction from 5.7'% to 1.0%), bitter beer (reduction from 3.6 to 0.7%) (L. Liguori, G. De Francesco, P. Russo, G. Perretti, D. Albanese, M. Di Matteo: Quality attributes of low-alcohol top-fermented beers produced by membrane contactor, Food and Bioprocess Technology, 9 (1) (2016), pp. 191-200) .

Pervaporation (PV) is a concentration-driven membrane-based process, which uses the principles of permeation and evaporation over a membrane. At first the liquid feed is contacted onto the membrane, without any additional pressure, at mild temperature (around 50 °C). The component that is intended to be separated will then interact with the membrane material due to the activity coefficient and thermo-dynamical affinity, and later permeates through the membrane, evaporates and leaves the membrane. The permeate stream may then be collected with the assistance of liquid nitrogen cold trap. A study used pervaporation with flat composite PDMS membrane for wine dealcoholization. The dealcoholization process was conducted at 40 °C and 1.3 kPa of permeate pressure. The process could produce wine with 3-7% of ethanol while the average flux was 1.5 kg m-2.h (S.-J. Tan, Z.-Y. Xiao, L. Li, F.-W. Wu, Z.-H. Xu, Z.-B. Zhang: Experimental research on dealcoholization of wine by pervaporation, Jingxi Huagong/Fine Chemicals, 20 (2) (2003), p. 69).

A membrane distillation using non-porous membrane which is similar to the pervaporation process, has been used by Indonesian scientists for the dealcoholization of Anker beer, a local Indonesian beer. The dealcoholization process was carried out by using non-porous spiral wound membrane from DOW Filmtec, composed of polyamide thin film composite membrane, at room temperature and 2-3 bar gauge. The permeate of alcohol was drawn by vacuum ranging from 0.49 to 0.66 bar. This process successfully reduced ethanol from 5% ABV to 2.45% ABV in 6 h, without significant loss of valuable nutritious components (maltose and glycerol) (M. Purwasasmita, D. Kurnia, F.C. Mandias, Khoiruddin, I.G. Wenten: Beer dealcoholization using non-porous membrane distillation, Food and Bioproducts Processing, 94 (2015), pp. 180-186).

Methods for dewatering alcoholic solutions via forward osmosis (FO) is disclosed in WO 2016/210337 A2. According to the prior art method, an alcoholic feed solution is introduced in a chamber in contact with a first side of a membrane and a draw solution having an ethanol concentration higher than the feed solution, is introduced in a chamber in contact with a second side of the membrane and water is transported from the feed side of the membrane to the draw solution. The application promises that the alcohol concentration can be increased two times or more and preferably five times or more in the feed. However, specific embodiments or examples are not provided in the application.

US 2010/0155333 discloses the use of forward osmosis for dewatering an aqueous ethanolic solution. The membrane has a water/ethanol selectivity greater than 1. In the examples, a cellulose triacetate membrane is used to dewater a 5 % by weight aqueous ethanol solution to 50 weight percent.

The various technologies used for dealcoholization of alcoholic beverages applies the principle of ethanol migration through the membrane. In a forward osmosis setting the present inventors therefore also expected the ethanol to migrate through the membrane, thereby depleting ethanol from the ethanolic feed solution. To their surprise, the inventors discovered that ethanol was rejected by the membrane, whereas water was allowed to penetrate the membrane, thus enriching the original ethanolic feed solution in ethanol. On this background, it is an object of the present invention to devise a method for enriching an aqueous ethanolic solution in ethanol.

### SUMMARY

It is an object of the present invention to provide a method for enriching an aqueous ethanolic solution in ethanol, comprising the steps of:
a. Providing a forward osmosis membrane module comprising a first chamber, a second chamber, and a semi-permeable membrane separating the first and the second chamber,
b. Coupling an inlet of the first chamber fluidly to a source of an aqueous ethanolic solution,
c. Coupling an inlet of the second chamber fluidly to a source for a concentrated draw solution, and
d. Recovering an aqueous ethanolic solution enriched in ethanol at an outlet of the first chamber and a diluted draw solution at the outlet of the second chamber, wherein the forward osmosis membrane module is a hollow fiber (HF) module and the semi-permeable membrane comprises a support layer covered by a Thin Film Composite (TFC) layer, and aquaporin water channels are incorporated into the TFC layer of the semipermeable membrane, and wherein the TFC layer is obtained by interfacial polymerization of a polyfunctional amine and a polyfunctional acyl halide.

The semipermeable membrane comprises a TFC layer and a supporting substrate layer. The supporting layer is a porous substrate, e.g. a nanoporous or microporous layer. In some case, the porous support layer may further be reinforced by being casted on a woven or non-woven sheet, e.g. formed from polyester fibers. The porous substrate is generally prepared of polyethersulfone (PES), polysulfone (PS), polyphenylene sulfone, polyether imide, polyvinylpyrrolidone and polyacrylonitrile, including blends and mixtures thereof. The support substrate layer is modified by forming a thin film composite (TFC) layer through interfacial polymerization. The TFC layer is prepared using a polyfunctional amine reactant, preferably an aromatic amine, such as a diamine or triamine, e.g. 1,3-diaminobenzene (m-Phenylenediamine - MPD) in an aqueous solution, and a polyfunctional acyl halide, i.e., an acyl halide reactant, such as a di- or triacid chloride, preferably an aromatic acyl halide, e.g. benzene-1,3,5-tricarbonyl chloride (TMC) dissolved in an organic solvent where said reactants are combined in an interfacial polymerization reaction.

While the rejection of ethanol is expected to apply for any semi-permeable membrane described above and capable of performing a forward osmosis process, the water flux becomes more efficient when aquaporin water channels are incorporated into the semipermeable membrane. Aquaporin water channels are transmembrane proteins widely occurring in nature for selective transportation of water in or out of cells. In an industrial setting, the aquaporin water channels in a semi-permeable membrane ensure the flow of water by osmosis, while other solutes in the solution are rejected. The presence of active aquaporin water channels thus assists the semi-permeable membrane rejecting ethanol and in promoting the penetration of water through the membrane.

The semi-permeable membrane comprises a TFC layer incorporating aquaporin water channels and a support layer. The aquaporin water channels are incorporated in the membrane in the active confirmation for at least a significant amount of the molecules. According to an aspect of the invention, the activity of the aquaporin water channels is maintained when the aquaporin water channels are assembled in a nanostructure comprising polyalkyleneimine, such as polyethyleneimine. As explained in further detail in WO17137361, polyalkyleneimine, such as polyethyleneimine (PEI), form self-assembled nanostructures with transmembrane proteins, such as aquaporin water channels. The nanostructures ensure that at least a part of the aquaporin water channels remain active even after incorporation into the TFC layer. It is currently believed that the polymer interacts with the transmembrane protein to prevent it from reacting with monomers participating in the formation of a TFC layer.

Generally, the PEI is a substantially linear or branched polymer having an average molecular weight of between about 2,000 Da to about 10,000 Da, such as between about 3,000 Da to about 5,000 Da. It is currently believed that the relatively short polymer interacts with the transmembrane protein to prevent it from reacting with monomers participating in the formation of a TFC layer, while at the same time not substantially inhibiting the interaction with water.

To prevent aggregation of aquaporin water channels, it may be an advantage to have the aquaporin water channel solubilized in a detergent prior to the assembling in a nanostructure comprising polyalkyleneimine. Due to the natural occurrence of the aquaporin water channel in the cell membrane, the protein displays a hydrophobic domain. It is believed that the hydrophobic domain of a detergent interacts with the hydrophobic domain of the aquaporin water channel, thereby forming a solubilized protein. The aquaporin water channel may be solubilized by a number of detergents.

In another aspect of the current invention the aquaporin water channels are provided in a vesicle prior to the incorporation in the TFC layer. Vesicles are the natural environment for the aquaporin water channels and the vesicles may be formed by a number of different membrane forming materials including the naturally occurring phospholipids. In a certain embodiment of the invention the vesicle is formed of an amphiphilic diblock copolymer, such as poly(2-methyloxazoline)-block-poly(dimethyl siloxane) diblock copolymer (PMOXA-PDMS) and a reactive end group functionalized poly(dimethyl siloxane) (PDMS).

The two blocks of the PMOXA-PDMS diblock co-polymer may be different lengths. To obtain sufficient stability of the vesicle the PMOXA-PDMS diblock co-polymer is typically selected from the group consisting of PMOXA₁₀₋₄₀-PDMS₂₅₋₇₀ and mixtures thereof. Experiments have shown that a mixture of different PMOXA-PDMS diblock co-polymers shows higher robustness..

The reactive end group functionalised PDMS (reactive end group functionalized poly(dimethyl siloxane)) of the vesicle may be functionalized with one or more of amine, carboxylic acid, and/or hydroxy groups. In a certain aspect of the invention the reactive end group functionalised PDMS_{e-f} is bis(amino alkyl), bis(hydroxyalkyl), or bis(carboxylic acid alkyl) terminated PDMS_{e-f}, such as poly(dimethyl siloxane), or poly(dimethyl siloxane), bis(3-hyroxypropyl). Suitably, the integer e is selected in the range of 20 to 40, such as 30 and the integer f is selected from the range of 40 to 80, such as 50. Prior to the incorporation of the vesicles with aquaporin water channels, the vesicles may be present in a liquid composition.

The vesicle of the invention may further contain about 1 % v/v to about 12 % v/v of triblock copolymer of the PMOXA_{a-b}-PDMS_{c-d}-PMOXA_{a-b} type to increase its integrity. Typically, said vesicle comprises from about 8 % v/v to about 12 % v/v of triblock copolymer of the PMOXA_{a-b}-PDMS_{c-d}-PMOXA_{a-b} type.

The vesicle of the invention may further comprise a flux improving agent to increase either the water flux or decrease the reverse salt flux. The flux improving agent is selected from alkylene glycol monoalkyl ether alkylate, beta cyclodextrin, or polyethylene glycol (15)-hydroxy stearate.

The vesicle of the invention may be present in a liquid composition before immobilization in a membrane, such as a TFC layer provided on a support membrane. Before the aquaporin water channels are mixed with the other constituents. Without wishing to be bound by any particular theory, it is believed that the vesicles containing free available reactive groups on the surface will be not only physically incorporated or immobilised in (adsorbed), but, in addition, chemically bound in the TFC layer, because the reactive free end groups, such as amino groups, hydroxyl groups and carboxyl groups, will participate in the interfacial polymerization reaction with the acyl chloride, such as a trimesoyl chloride (TMC). In this way, it is believed that vesicles will be covalently bound in the TFC layer, leading to relatively higher vesicle loading and thus higher water flux through the membranes. In addition, it is believed that the covalent coupling of vesicles in the TFC layer results in higher stability and/or longevity of the aquaporin water channels and the vesicles containing aquaporin water channels when incorporated in the selective membrane layer.

The vesicles may be prepared in a liquid composition incorporating the aquaporin water channels, comprising the step of stirring a mixture of a solution of an amphiphilic diblock copolymer of the PMOXA_{a-b}-PDMS_{c-d} type, 0.05% to about 1% of reactive end group functionalised PDMS_{e-f}, and aquaporin water channels. To obtain the best result, the stirring is continued for 12-16 hours.

The preparation of a thin film composite layer immobilizing vesicles incorporating the aquaporin water channels on a porous substrate membrane comprises the steps of providing a mixture of vesicles in a liquid composition prepared as disclosed above, and a di-amine or tri-amine compound, covering the surface of a porous support membrane with the mixture, applying a hydrophobic solution comprising an acyl halide compound, and allowing the aqueous solution and the hydrophobic solution to perform an interfacial polymerization reaction to form the thin film composite layer. In a certain embodiment of the invention, the hydrophobic solution further comprises a TFC layer modifying agent in an amount of 0.1 to 10% by volume. The TFC layer modifying agent has the purpose to increase the water flow and/or the rejection of solutes. In a suitable embodiment, the TFC layer modifying agent is a C3 to C8 carbonyl compound. As an example, the TFC layer modifying agent is selected among the group consisting of diethylene ketone, 2-pentanone, 5-pentanone, and/or cyclopentanone.

In a preferred aspect the diamine is selected as *m-*phenylenediamine (MPD) also known as 1,3-diaminobenzene. The tri-amine compound may be selected among a range of compounds including for example, diethylene triamine, dipropylene triamine, phenylenetriamine, bis(hexamethylene)triamine, bis(hexamethylene)triamine, bis(3-aminopropyl)amine, hexamethylenediamine, N-tallowalkyl dipropylene, 1,3,5-triazine-2,4,6-triamine, and mixtures of these compounds.

The acyl halide compound usually has two or three acyl halide groups available for reaction with the di- or triamine compound. Suitable examples of diacyl halide or triacyl halide compounds include trimesoyl chloride (TMC), trimesoyl bromide, isophthaloyl chloride (IPC), isophthaloyl bromide, terephthaloyl chloride (TPC), terephthaloyl bromide, adipoyl chloride, cyanuric chloride and mixtures of these compounds.

The amine groups of the di-amine or tri-amine compound will compete with the acid chloride groups of the acyl halide compound for reaction. Generally, the proportion by weight of the di-amine or tri-amine compound to acyl halide compound is from 0:1 to 30:1. When a high density of vesicles on the surface is required the amount of di-amine or tri-amine groups is usually in the lower part of the range, i.e. 0:1 to 1:1, such as between 0:1 to 0.5:1. In other embodiments of the invention, a more rigid TFC layer is desired and a selection of the reactants are in the higher end of the range, such as 1:1 to 30:1, preferably 1:1 to 5:1.

The porous support membrane may be a hollow fiber membrane. The hollow fiber membrane is generally preferred, as it provides for higher packing density, i.e. the active membrane area is higher. The membranes may be grouped together or assembled into a module as known in the art.

The hollow fiber membranes may be assembled into a module by assembling a bundle of hollow fibers in a housing, wherein an inlet for passing a first solution is connected to the lumen of the hollow fibers in one end and an outlet is connected to the lumen in the other end, and an inlet is provided in the housing for passing a second solution to an outlet connected to the housing. Hollow fiber elements utilize cross flow technology, and because of its construction, can easily be created in different configurations with varying length, diameter, and membrane material.

The aqueous ethanolic solution may be used as the first or the second solution, i.e. the aqueous ethanolic solution may be directed through the bore of the hollow fiber or may be directed to the exterior of the fibers. In a currently preferred embodiment of the invention the position of the TFC layer on either the inside or the outside of the fibers determines to which compartment of the hollow fiber module the aqueous ethanolic solution is directed. Thus, in a certain embodiment, the TFC layer of the membrane is facing the aqueous ethanolic solution to be enriched to obtain a less complicated process as components of the aqueous ethanolic solution may occlude the pores in the support membrane.

While there may be certain advantages of providing the TFC layer on the outside of the fibers, such as a higher membrane area per module volume, it is currently preferred to use a forward osmosis module in which the TFC layer is provided on the lumen side of the hollow fibers. Using this configuration, the aqueous ethanolic solution is fed to the inlet fluidly connected to the lumen of the fibers and a solution enriched in ethanol is recovered at the outlet fluidly connected to the other end of the hollow fibers.

A hollow fiber module having a TCF layer on the inside of the fiber may be prepared by entering the aqueous phase containing the polyfunctional amine such as MPD into the lumen of the substrate fibers. After allowing the substrate fibers to soak the aqueous phase, the surplus liquid is drained or purged with air out of the lumen. Subsequently, a mild vacuum may be applied to the outside of the fibers, i.e. the shell side, to promote uniform drying of the aqueous phase. An organic polyfunctional acyl halide such as TMC may then be entered into the lumen of the fibers. The reaction between the polyfunctional amine and the polyfunctional acyl halide occurs almost instantly and forms the TFC layer. The TFC layer is relatively dense due to a high degree of cross-linking and thin, providing for a high selectivity relative to ethanol and a high water flux.

It is currently believed that the deposition of the polyfunctional amine in a thin layer after to the vacuum drying step, accounts for the observed dense and thin TFC layer, due to a limited diffusion of the polyfunctional amine during the reaction with the polyfunctional acyl halide. In an aspect of the invention the skin layer part (i.e. the active layer) of the TFC layer has a thickness of 200 nm or less, such as 150 nm or less, and suitably 120 nm or less. To ensure sufficient durability of the active layer the thickness is suitably not less than 50 nm. In a preferred aspect, the thickness of the active layer is between 80 nm and 110nm.

The draw solution is fed to the opposite chamber as the aqueous ethanolic solution. Thus, if the aqueous ethanolic solution is sourced to the lumen of the fibers of the hollow fiber module, then the draw solution is sourced to the space surrounding the exterior of the fibers. Conversely, if the aqueous ethanolic solution is sourced to the space surrounding the exterior the fibers, then the draw solution is sourced to the lumen of the fibers.

The draw solution comprises one or more dissolved aqueous solutes. The concentration of the solutes provides for an osmotic pressure difference between the first and the second chamber, drawing water molecules from the aqueous ethanolic solution to the draw solution. The solute may be the selected from a variety of different molecules depending i.a. of the nature of the aqueous ethanolic solution and the use of the solution enriched in ethanol. Suitable examples of solutes include salts like NaCl, MgCl₂, sodium citrate, and sodium acetate. Other suitable solutes include carbohydrates like glucose, sucrose, fructose, glycerol, etc. As a minor amount of the solutes may penetrate the membrane, care should be taken in the selection of the solute when the final product is intended for human or animal consumption.

The concentration of the solute may vary in dependency of the osmolarity of the aqueous ethanolic solution, the use of the end product, etc. Thus, in a certain embodiment of the invention, the concentration of the solute in the draw solution is at least 0.2 M, such as at least 0.5 M such as at least 1 M and suitably at least 1.5 M. Alternatively, the osmotic pressure created by the solute may be at least 10 bar, such as at least 20 bar, suitably at least 30, and preferably at least 50 bar.

The aqueous ethanolic solution source may be recirculated until it has been observed that a certain amount of the matter has been recovered in the diluted draw solution or, alternatively, disappeared from the feed aqueous ethanolic solution. In a preferred aspect of the invention, the aqueous ethanolic solution is recirculated between the outlet and the inlet of the first chamber until at least 50%, such as at least 60%, such as at least 70%, such as at least 80% and preferably at least 85% of the weight of the original aqueous ethanolic solution has been recovered in the draw solution.

The concentration of ethanol in the aqueous ethanolic solution may be at least 2% vol/vol, such as at least 4% vol/vol. Usually, the concentration of ethanol in the aqueous ethanolic solution is less than 20% vol/vol, such as less than 15% vol/vol. In an embodiment of the invention the aqueous ethanolic solution contains an essentially pure mixture of ethanol and water. In another embodiment of the invention, the aqueous ethanolic solution is beer or ale.

When beer or wine is transported over large distances the amount of water is crucial for the freight costs. Therefore, reduction of the water content in beer or wine can be translated into lower transport expenses. When the wine or beer enriched in ethanol reaches its destination, it may be supplemented with water to reach the original ethanol concentration. Ideally, beer or ale enriched in ethanol maintains the distinctive flavor and taste as only water is removed from the source. In practice however, a minor reverse flux of solutes also occurs, which may slightly alter the original taste after dilution. Therefore, it may be desired or needed to supply the ethanol enriched solution with solutes in addition to water when the original product is reconstituted.

Another reason for enriching a beer or wine in ethanol is that the product becomes better preserved since ethanol is a natural antiseptic. Furthermore, the aging of wines and beers tend to slow down when the amount of ethanol is increased, thereby increasing the shelf life and protecting the wine or beer from deterioration due to temperature variations.

The alcoholic beverages enriched in ethanol may be a consumer product in its own right. Thus, organoleptic tests suggest that a pilsner type beer enriched in ethanol resembles a barley wine in taste and flavor. Wine enriched in ethanol may be brought on the market along with other ethanol fortified wines like port wine, sherry, madeira etc.

The type of beer which may be treated by the method according to the present invention is not particular limited an in principle include any beer type, including altbier, amber ale, Berliner weisse, bitter, biére de garde, blonde ale, bock, brown ale, California common/steam beer, cream ale, dopplebock, Dortmunder export, dunkel, dunkelweizen, eisbock, Flanders red ale, golden/summer ale, Gose, Gueuze, Hefeweizen, Helles, India pale ale, Kölsch, Lambic, light ale, Maibock/Helles bock, malt liquor, mild, Oktoberfestbier/Märzenbier, old ale, oud bruin, pale ale, Pils/pilsner, porter, red ale, roggenbier, saison, Schwartzbier, Scotch ale, stout, Vienna lager, Weissbier, Weizenbock, witbier, etc. The present invention is notably suitable for beer or ale having a strength below 7% vol/vol, such as below 6% vol/vol, and preferably below 5% vol/vol, as beer having a strength in the lower end has a tendency to degrade faster after production. Suitably, the strength of the beer or ale is more than 2% vol/vol, preferably more than 3% vol/vol.

The method of the present invention may also be applied as a pre-step to distillation. Ethanol distillation from a fermented broth is a traditional way of enriching a solution in ethanol. If the desired ethanol concentration of the end product is higher than what the method according to present invention can provide, it can be followed by a traditional distillation process.

### Example 1

### Ethanol enrichment of beer

A HFFO2/220 element available from Aquaporin, Denmark, was used in this experiment for concentrating ethanol in beer. Initially, the membrane was flushed for 30 min with DI water and stored at 4°C.

40 kg of Royal Classical Pilsner available from Royal Unibrew, Denmark was continuously conveyed through the lumen of the hollow fibers using a gear pump adjusted to a volumetric velocity of 60 L/h at the outlet of the module. The initial ethanol concentration in the beer was 4.6 vol% corresponding to 31.55 g/L. A draw solution of 2M MgCl₂ was delivered in continuous mode and adjusted by a gear pump to a volumetric velocity of 25 L/h at the outlet of the module.

The weight increase of the draw solution was used to calculate the recovery rate, i.e. the amount of matter exchanged between the beer and the draw solution, and the flux. The initial flux was measured as 10,24 LMH.

After recovery of 87% of the feed mass in the draw solution a sample was collected and analyzed. The analysis showed that the amount of ethanol in the feed solution was 165.65 g/L corresponding to the ethanol rejection being 90.43%.

### Example 2

### Alcohol enrichment of alcoholic aqueous solution

A HFFO2/220 element available from Aquaporin, Denmark, was used in this experiment for concentrating ethanol in an aqueous ethanolic solution. Initially, the membrane was flushed for 30 min with DI water and stored at 4°C.

28.6 kg of alcoholic aqueous solution was continuously conveyed through the lumen of the hollow fibers using a gear pump adjusted to a volumetric velocity of 60 L/h at the outlet of the module. The initial ethanol concentration in the solution was 5 vol% corresponding to 32.15 g/L. A draw solution of 2M MgCL₂ was delivered to the module in continuous mode and adjusted by a gear pump to a volumetric velocity of 25 L/h at the outlet of the module.

The weight increase of the draw solution was used to calculate the recovery rate, i.e. the amount of matter exchanged between the aqueous ethanolic solution and the draw solution, and the flux. The initial flux was measured as 14.87 LMH.

After recovery of 83.8% of the feed mass in the draw solution a sample was collected and analyzed. The analysis showed that the amount of ethanol in the feed solution was 205.27 g/L corresponding to the ethanol rejection being 68.08%.

### Example 3

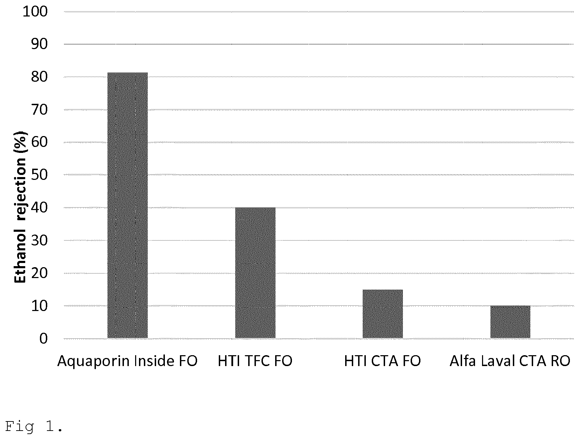

The HFFO2/220 element (Aquaporin Inside^{®} FO) was compared with other commercially available forward osmosis membranes. The results are shown in Figure 1.

HTI TFC FO is a membrane available from Hydration Technology Innovations (HTI) under the trademark OsMEM^{™}. The membrane is of the flat sheet type having a Thin Film Composite (TFC) layer. The flat sheet membrane is provided on a durable woven backing and spiraled into a module. The NaCl rejection is specified to 99.4%.

HTI CTA FO is a membrane available from Hydration Technology Innovations (HTI) under the trademark OsMEM^{™}. The membrane is produced of cellulose triacetate and provided with a woven backing. The membrane and the backing are spiral wound to produce the membrane module.

Alfa Laval CTA RO is a spiral wound membrane module produced from a flat sheet membrane of cellulose triacetate.

For the FO experiments, the feed solution was an aqueous ethanolic solution of 4.5% EtOH in water and the draw solution was 2M MgCl₂ for the HFFO2/220 element, whereas 1 M NaCl was used for the HTI elements. For the RO experiment, a beer having an ethanol concentration of 5.5% was used as the feed solution and 30 bar was applied as the feed pressure.

Surprisingly, the data in Fig. 1 shows that a higher ethanol rejection is obtained by the HFFO2 hollow fiber module.

### Example 4

To investigate the results of example 3 further, a hollow fiber module was prepared without aquaporins in the TFC layer of the membrane. The purpose was to find out if it was the presence of aquaporins in the membrane that accounted for the ethanol rejection.

The hollow fiber module was prepared as disclosed in WO2017137361, however without adding PEI-APQ Z to the MPD (m-phenylene diamine) solution. More specifically, MPD was dissolved in MilliQ water in a concentration of 2.5%(w/w). The MPD solution was filled into the lumen of the fibers in a UF hollow fiber module having a total membrane area of 2.2 m² at a flow rate of 5 mL/min. After 1 min the flow was stopped and the fibers were left for soaking for 1 min. Then, the module was emptied and purge with air to get surplus MPD solution out. To remove surface water from the lumen of the fibers an air flow was used having a flow rate of 25L/min. Subsequently, a mild vacuum was applied to the shell side of the module to promote uniform drying of the aqueous phase.

A TMC solution was prepared by dissolving benzene-1,3,5-tricarbonyl chloride (Trimesoyl Chloride - TMC) in hexane to obtain a final concentration of 0.25% (w/v). The TMC solution was pumped into the module using a flow rate of 15 mL/min. After the module was filled the pumping was continued for 30s. Subsequently, the module was turned upside down to empty it for free-flowing liquid. Then the module was connected to air and purged at 10 L/min for 5-10 s. Finally, the lumen of the fibers in the membrane module was rinsed with MilliQ water. The active layer of the TMC layer was measure to between 83 nm to 104 nm in a SEM image.

The hollow fiber module without aquaporins in the TFC layer but otherwise similar to the HFFO2 module is termed HFFO2 (-AQP). The HFFO2 module and the HFFO2 (-AQP) were tested on a feed solution comprising 5% EtOH in RO water at a flow rate of 1000 mL/min in batch mode. The draw solution was 2 M or 2.4 M MgCl₂ and supplied to the module at a flow rate of 416 mL/min. Each test was performed twice (n=2) to obtain statistical data samples.

The data show that after about 50% recovery the HFFO2 module and the HFFO2 (-AQP) had a similar ethanol rejection. The same tendency appeared after about 80 % and about 90 % recovery. The water flux was at a similar level for the HFFO2 module and the HFFO2 (-AQP) at 50% recovery. However, after about 80 % and about 90 % recovery the water flux was substantially higher for the HFFO2 module. In short, the presence or absence of aquaporins in the TFC layer do not substantially affects the ethanol rejection property. The water flux is, however, substantially higher for the hollow fiber module comprising aquaporins.

## Claims

1. A method for enriching an aqueous ethanolic solution in ethanol, comprising the steps of:
a. Providing a forward osmosis membrane module comprising a first chamber, a second chamber and a semi-permeable membrane separating the first and the second chamber,
b. Coupling an inlet of the first chamber fluidly to a source of an aqueous ethanolic solution,
c. Coupling an inlet of the second chamber fluidly to a source for a concentrated draw solution, and
d. Recovering an aqueous ethanolic solution enriched in ethanol at an outlet of the first chamber and a diluted draw solution at the outlet of the second chamber,
wherein the forward osmosis membrane module is a hollow fiber (HF) module, the semi-permeable membrane comprises a support layer covered by a Thin Film Composite (TFC) layer, and aquaporin water channels are incorporated into the TFC layer of the semipermeable membrane, and
wherein the TFC layer is obtained by interfacial polymerization of a polyfunctional amine and a polyfunctional acyl halide.

2. The method according to claim 1, wherein the TFC layer of the membrane is facing the aqueous ethanolic solution to be enriched.

3. The method according to claim 1 or 2, wherein the TFC layer is present on the inside of the fibers and the lumen of the hollow fibers constitutes the first chamber and the second chamber is constituted by the space around the exterior of the fibers.

4. The method according to anyone of the preceding claims, wherein the aqueous ethanolic solution is treated in the forward osmosis membrane module until at least 50% of the weight thereof has been recovered in the draw solution.

5. The method according to anyone of the preceding claims, wherein the concentration of ethanol in the starting aqueous ethanolic solution is less than 20%.

6. The method according to anyone of the preceding claims, wherein the aqueous ethanolic solution is beer, ale, or wine.

7. The method according to anyone of the preceding claims, wherein the aquaporin water channels are assembled in a nanostructure comprising polyalkyleneimine.

8. The method according to anyone of the preceding claims, wherein the polyalkyleneimine is a polyethyleneimine that has an average molecular weight of between about 2,000 Da to about 10,000 Da.

9. The method according to claim 7 or 8, wherein the aquaporin water channel is solubilized in a detergent prior to the assembling in a nanostructure comprising polyalkyleneimine.

10. The method according to anyone of the preceding claims 1 to 6, wherein the aquaporin water channels are provided in a vesicle prior to the incorporation in the TFC layer.

11. The method according to claim 10, wherein the vesicle comprises an amphiphilic diblock copolymer of the poly(2-methyloxazoline)-block-poly(dimethyl siloxane) diblock copolymer (PMOXA-PDMS) type and a reactive end group functionalized poly(dimethyl siloxane) (PDMS).

12. The method according to claim 11, wherein said PMOXA-PDMS is selected from the group consisting of PMOXA₁₀₋₄₀-PDMS₂₅₋₇₀ and mixtures thereof.

13. The method according to claim 11 or 12, wherein said reactive end group functionalised PDMS is functionalized with one or more of amine, carboxylic acid, and/or hydroxy groups.

14. The method according to anyone of the claims 11 to 13, wherein the vesicle further comprises from about 1% v/v to about 12% v/v of triblock copolymer of the PMOXA-PDMS-PMOXA type.

15. The method according to anyone of the claims 11 to 14, wherein the vesicle further comprises a flux improving agent that either increases the water flux or decreases the reverse salt flux.

## Patentansprüche

1. Verfahren zum Anreichern einer wässrigen ethanolischen Lösung in Ethanol, umfassend die folgenden Schritte
a. Bereitstellen eines Vorwärtsosmosemembranmoduls, das eine erste Kammer, eine zweite Kammer und eine semipermeable Membran, die die erste und die zweite Kammer voneinander trennt, umfasst,
b. Ankoppeln eines Einlasses der ersten Kammer fluidisch an eine Quelle einer wässrigen ethanolischen Lösung,
c. Ankoppeln eines Einlasses der zweiten Kammer fluidisch an eine Quelle für eine konzentrierte Entnahmelösung, und
d. Gewinnen einer wässrigen ethanolischen Lösung, die mit Ethanol angereichert ist, an einem Auslass der ersten Kammer und einer verdünnten Entnahmelösung am Auslass der zweiten Kammer,
wobei das Vorwärtsosmosemembranmodul ein Hohlfasermodul (HF) ist, die semipermeable Membran eine Trägerschicht umfasst, die von einer Dünnfilm-Verbundschicht (TFC) bedeckt ist, und Aquaporinwasserkanäle in die TFC-Schicht der semipermeablen Membran integriert sind, und
wobei die TFC-Schicht durch Grenzflächenpolymerisation eines polyfunktionellen Amins und eines polyfunktionellen Acylhalogenids erhalten wird.

2. Verfahren nach Anspruch 1, wobei die TFC-Schicht der Membran der anzureichernden wässrigen ethanolischen Lösung zugewandt ist.

3. Verfahren nach Anspruch 1 oder 2, wobei die TFC-Schicht auf der Innenseite der Fasern vorhanden ist und das Lumen der Hohlfasern die erste Kammer bildet und die zweite Kammer durch den Raum um die Außenseite der Fasern gebildet wird.

4. Verfahren nach einem der vorhergehenden Ansprüche, wobei die wässrige ethanolische Lösung in dem Vorwärtsosmosemembranmodul behandelt wird, bis mindestens 50 % ihres Gewichts in der Entnahmelösung wiedergewonnen wurden.

5. Verfahren nach einem der vorhergehenden Ansprüche, wobei die Konzentration von Ethanol in der wässrigen ethanolischen Ausgangslösung weniger als 20 % beträgt.

6. Verfahren nach einem der vorhergehenden Ansprüche, wobei die wässrige ethanolische Lösung Bier, Bier oder Wein ist.

7. Verfahren nach einem der vorhergehenden Ansprüche, wobei die Aquaporinwasserkanäle in einer Nanostruktur angeordnet sind, die Polyalkylenimin umfasst.

8. Verfahren nach einem der vorhergehenden Ansprüche, wobei das Polyalkylenimin ein Polyethylenimin ist, das ein durchschnittliches Molekulargewicht zwischen etwa 2.000 Da und etwa 10.000 Da aufweist.

9. Verfahren nach Anspruch 7 oder 8, wobei der Aquaporinwasserkanal vor dem Zusammenbau in einer Nanostruktur, die Polyalkylenimin umfasst, in einem Detergens solubilisiert wird.

10. Verfahren nach einem der vorhergehenden Ansprüche 1 bis 6, wobei die Aquaporinwasserkanäle in einem Vesikel vor dem Einbau in die TFC-Schicht bereitgestellt werden.

11. Verfahren nach Anspruch 10, wobei das Vesikel ein amphiphiles Diblockcopolymer vom Typ Poly(2-methyloxazolin)-Block-Poly(dimethylsiloxan) -Diblockcopolymer (PMOXA-PDMS) und ein reaktives Endgruppen-funktionalisiertes Poly (dimethylsiloxan) (PDMS) umfasst.

12. Verfahren nach Anspruch 11, wobei das PMOXA-PDMS ausgewählt ist aus der Gruppe, bestehend aus PMOXA₁₀₋₄₀-PDMS₂₅₋₇₀ und Mischungen davon.

13. Verfahren nach Anspruch 11 oder 12, wobei das reaktive endgruppenfunktionalisierte PDMS mit einer oder mehreren von Amin-, Carbonsäure- und/oder Hydroxygruppen funktionalisiert ist.

14. Verfahren nach einem der Ansprüche 11 bis 13, wobei das Vesikel ferner etwa 1 % v/v bis etwa 12 % v/v eines Triblockcopolymer des PMOXA-PDMS-PMOXA-Typs umfasst.

15. Verfahren nach einem der Ansprüche 11 bis 14, wobei das Vesikel ferner ein Flussverbesserungsmittel umfasst, das entweder den Wasserfluss erhöht oder den umgekehrten Salzfluss verringert.

## Revendications

1. Procédé pour enrichir une solution éthanolique aqueuse dans de l'éthanol, comprenant les étapes consistant à :
a. fournir un module de membrane d'osmose directe comprenant une première chambre, une seconde chambre et une membrane semi-perméable séparant la première et la seconde chambre,
b. coupler fluidiquement une entrée de la première chambre à une source d'une solution éthanolique aqueuse,
c. coupler fluidiquement une entrée de la seconde chambre à une source de solution d'extraction concentrée, et
d. récupérer une solution éthanolique aqueuse enrichie en éthanol à une sortie de la première chambre et d'une solution d'extraction diluée à la sortie de la seconde chambre,
dans lequel le module de membrane d'osmose directe est un module de fibre creuse (HF), la membrane semi-perméable comprend une couche de support recouverte d'une couche de composite à film mince (TFC), et des canaux d'eau d'aquaporine sont incorporés dans la couche de TFC de la membrane semi-perméable, et
dans lequel la couche de TFC est obtenue par polymérisation interfaciale d'une amine polyfonctionnelle et d'un halogénure d'acyle polyfonctionnel.

2. Procédé selon la revendication 1, dans lequel la couche de TFC de la membrane fait face à la solution éthanolique aqueuse à enrichir.

3. Procédé selon la revendication 1 ou 2, dans lequel la couche de TFC est présente à l'intérieur des fibres et la lumière des fibres creuses constitue la première chambre et la seconde chambre est constituée par l'espace autour de l'extérieur des fibres.

4. Procédé selon l'une quelconque des revendications précédentes, dans lequel la solution éthanolique aqueuse est traitée dans le module de membrane d'osmose directe jusqu'à ce qu'au moins 50 % de son poids ait été récupéré dans la solution d'extraction.

5. Procédé selon l'une quelconque des revendications précédentes, dans lequel la concentration d'éthanol dans la solution éthanolique aqueuse de départ est inférieure à 20 %.

6. Procédé selon l'une quelconque des revendications précédentes, dans lequel la solution éthanolique aqueuse est de la bière, de l'ale ou du vin.

7. Procédé selon l'une quelconque des revendications précédentes, dans lequel les canaux d'eau d'aquaporine sont assemblés dans une nanostructure comprenant de la polyalkylèneimine.

8. Procédé selon l'une quelconque des revendications précédentes, dans lequel la polyalkylèneimine est une polyéthylèneimine qui a un poids moléculaire moyen compris entre environ 2 000 Da et environ 10 000 Da.

9. Procédé selon la revendication 7 ou 8, dans lequel le canal d'eau d'aquaporine est solubilisé dans un détergent avant l'assemblage dans une nanostructure comprenant de la polyalkylèneimine.

10. Procédé selon l'une quelconque des revendications précédentes 1 à 6, dans lequel les canaux d'eau d'aquaporine sont prévus dans une vésicule avant l'incorporation dans la couche de TFC.

11. Procédé selon la revendication 10, dans lequel la vésicule comprend un copolymère dibloc amphiphile du type copolymère dibloc poly(2-méthyloxazoline)-bloc-poly(diméthyl siloxane) (PMOXA-PDMS) et un groupe terminal réactif fonctionnalisé poly(diméthyl siloxane) (PDMS).

12. Procédé selon la revendication 11, dans lequel ledit PMOXA-PDMS est choisi dans le groupe constitué par le PMOXA₁₀₋₄₀-PDMS₂₅₋₇₀ et des mélanges de ceux-ci.

13. Procédé selon la revendication 11 ou 12, dans lequel ledit PDMS fonctionnalisé par un groupe terminal réactif est fonctionnalisé avec un ou plusieurs groupes parmi les groupes amine, acide carboxylique et/ou hydroxy.

14. Procédé selon l'une quelconque des revendications 11 à 13, dans lequel la vésicule comprend en outre d'environ 1 % v/v à environ 12 % v/v de copolymère tribloc du type PMOXA-PDMS-PMOXA.

15. Procédé selon l'une quelconque des revendications 11 à 14, dans lequel la vésicule comprend en outre un agent améliorant le flux qui augmente le flux d'eau ou diminue le flux de sel inverse.
